# EUROPÄISCHE PATENTSCHRIFT

(11) **EP 1 259 473 B1**
(45) Veröffentlichungstag und Bekanntmachung des Hinweises auf die Patenterteilung: **26.10.2005**
(21) Anmeldenummer: 01919355.6
(22) Anmeldetag: 28.02.2001
(51) Int. Cl.: C07C 51/60, C07C 53/50

(54) **VERFAHREN ZUR HERSTELLUNG VON CHLORCARBONSÄURECHLORIDEN**
METHOD FOR PRODUCING CHLOROCARBOXYLIC ACID CHLORIDES
PROCEDE DE PRODUCTION DE CHLORURES D'ACIDE CHLOROCARBOXYLIQUE

(30) Priorität: 03.03.2000 DE 10010595
(43) Veröffentlichungstag der Anmeldung: 27.11.2002
(73) Patentinhaber: BASF Aktiengesellschaft, 67056 Ludwigshafen (DE)
(72) Erfinder: STAMM, Armin, 55268 Nieder-Olm (DE); KNEUPER, Heinz-Josef, 67150 Niederkirchen (DE); HENKELMANN, Jochem, 68165 Mannheim (DE); WEBER, Theodor, 67063 Ludwigshafen (DE); BUSCH, Ralph, 67549 Worms (DE)
(86) Internationale Anmeldenummer: PCT/EP2001/002239
(87) Internationale Veröffentlichungsnummer: WO 2001/064614

(56) Entgegenhaltungen:
- EP-A- 0 583 589
- US-A- 4 764 309

## Beschreibung

Die Erfindung betrifft ein Verfahren zur Herstellung von Chlorcarbonsäurechloriden der Formel (I) 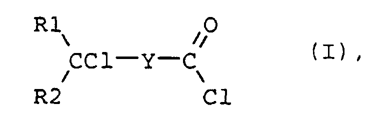 in der
R¹ und R² unabhängig voneinander
ein Wasserstoffatom, ein Kohlenstoff enthaltender organischer Rest, ein Halogen, eine Nitro- oder eine Cyanogruppe bedeuten,
und Y
eine Alkylenkette mit 1 bis 10 Kohlenstoffatomen in der Kette, die unsubstituiert oder durch Kohlenstoff enthaltende organische Reste, Halogen, Nitro- und/oder Cyanogruppen substituiert ist,
wobei die Alkylenkette durch eine Ether-, Thioether-, tertiäre Amino- oder Ketogruppe unterbrochen sein kann,
bedeutet,
wobei die Kohlenstoff enthaltenden organischen Reste von Y und/oder R¹ und/oder R² unter Bildung eines nicht-aromatischen Systems miteinander verbunden sein können,
durch Umsetzung eines Lactons der Formel (II) 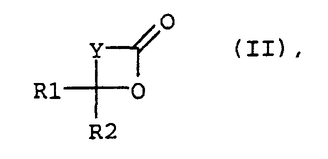 in der R¹, R² und Y die vorstehend genannte Bedeutung haben, mit Chlorwasserstoff und Phosgen in Gegenwart eines Katalysators.

Chlorcarbonsäurechloride sind wichtige reaktive Zwischenprodukte zur Herstellung pharmazeutischer und agrochemischer Wirkstoffe.

Chlorcarbonsäurechloride können zum Beispiel durch Umsetzung des entsprechenden Lactone mit Chlorierungsmitteln in Gegenwart eines Katalysators hergestellt werden. Als Chlorierungsmittel werden typischerweise Phosgen oder Thionylchlorid eingesetzt, da sie als Koppelprodukte ausschließlich gasförmige Substanzen (CO₂ bzw. SO₂ und HCl) bilden.

Bei der Verwendung von Phosgen als Chlorierungsmittel werden im allgemeinen verschiedene Katalysatorsysteme eingesetzt. Als geeignete Katalysatoren für die Phosgenierung von γ-Butyrolacton, γ- und δ-Valerolacton sind in US-Patent 2,778,852 Pyridine, tertiäre Amine, Schwermetalle und Säuren, wie Schwefelsäure, Phosphorsäure, Phosphorchlorid, Phosphoroxychlorid, Aluminiumchlorid, Sulfurylchlorid und Chlorsulfonsäure genannt.

Die Offenlegungsschrift DE-A 197 53 773 beschreibt die Phosgenierung aliphatischer Lactone in Gegenwart einer Harnstoffverbindung als Katalysator und gleichzeitiger Einleitung von Chlorwasserstoff.

In den Offenlegungsschriften EP-A 0 413 264 und EP-A 0 435 714 ist die Phosgenierung von Lactonen in Gegenwart eines Phosphinoxide als Katalysator offenbart, wobei nach der Lehre von EP-A 0 413 264 die gleichzeitige Einleitung von Chlorwasserstoff als vorteilhaft beschrieben ist.

Die Offenlegungsschrift EP-A 0 583 589 beschreibt die Phosgenierung benzokondensierter Lactone in Gegenwart einer organischen Stickstoffverbindung, wie etwa einem quartärem Ammoniumsalz, einem Amin, einem Stickstoffheterocyclus, einer Harnstoffverbindung, einer Guanidinverbindung oder einem Formamid als Katalysator und gleichzeitiger Einleitung von Chlorwasserstoff.

In der Offenlegungsschrift EP-A 0 253 214 ist die Phosgenierung aliphatischer Lactone in Gegenwart eines quartären Ammoniumsalzes als Katalysator, konkret Trimethylbenzylammoniumchlorid, N,N-Di-methylpiperidiniumchlorid und Dimethylmorpholiniumchlorid, genannt, wobei die gleichzeitige Einleitung von Chlorwasserstoff als besonders vorteilhaft beschrieben ist. Bei der Umsetzung von δ-Valerolacton mit Phosgen und Chlorwasserstoff bei 175 bis 180°C in Gegenwart von N,N-Dimethylpiperidiniumchlorid wurde 5-Chlorvaleriansäurechlorid mit einer Reinheit von 98,1% und einer Ausbeute von 76,2% erhalten.

Erfindungsgemäß wurde erkannt, daß bei der Herstellung von Chlorcarbonsäurechloriden, insbesondere 5-Chlorvaleriansäurechloriden, durch Phosgenierung der entsprechenden Lactone in Gegenwart der oben genannten Katalysatoren zum Teil erhebliche Probleme auftreten. Da die einzusetzenden Lactone, insbesondere die 6-Ring-Systeme, unter den üblichen Phosgenierungsbedingungen zum Teil sehr labil sind, wird unter den vorbeschriebenen Bedingungen vielfach ein hoher Anteil an Oligomeren gebildet, was sich in einem deutlichen Anstieg der Viskosität bis hin zum Erstarren der Reaktionsmischung äußert. Es besteht dadurch ein nicht unerhebliches Sicherheitsrisiko durch Verstopfung von Apparaten und Leitungen im Bereich der Synthese und der in der Regel destillativen Aufarbeitung. Durch die verstärkte Oligomerenbildung wird die Ausbeute an Wertprodukt signifikant verringert und der Anteil an entsorgungsbedürftigem Rückstand deutlich erhöht.

Als weiterer Nachteil bei der Anwendung üblicher Phosgenierungsbedingungen stellte sich heraus, daß die Reaktionsansätze in vielen Fällen sehr stark schäumen und die Reaktion dadurch deutlich zurückgefahren oder gar abgebrochen werden muß. Auch die vermehrte Schaumbildung stellt ein deutliches Sicherheitsrisiko dar.

Es bestand daher die Aufgabe, ein verfahren zur Herstellung von Chlorcarbonsäurechloriden durch Umsetzung der entsprechenden Lactone mit Chlorierungsmitteln zu entwickeln, welches die bekannten Nachteile nicht mehr besitzt, eine sichere Reaktionsführung ermöglicht und die Chlorcarbonsäurechloride in hoher Ausbeute und hoher Reinheit zugänglich macht.

Demgemäß wurde ein Verfahren zur Herstellung von Chlorcarbonsäurechloriden der Formel (I) 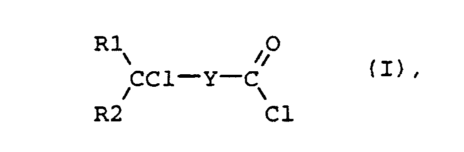 in der
R¹ und R² unabhängig voneinander
ein Wasserstoffatom, ein unsubstituierter oder substituierter, aliphatischer, aromatischer, oder araliphatischer Rest mit 1 bis 20 Kohlenstoffatomen, ein Halogen, eine Nitro- oder eine Cyanogruppe bedeuten,
und Y
eine Alkylenkette mit 1 bis 10 Kohlenstoffatomen in der Kette, die unsubstituiert oder durch unsubstituierte oder substituierte, aliphatische, aromatische oder araliphatische Reste mit 1 bis 20 Kohlenstoffatomen, Halogen, Nitro- und/oder Cyanogruppen substituiert ist, wobei die Alkylenkette durch eine Ether-, Thioether-, tertiäre Amino- oder Ketogruppe unterbrochen sein kann,
bedeutet,
wobei die genannten unsubstituierten oder substituierten aliphatischen, aromatischen oder araliphatischen Reste mit 1 bis 20 Kohlenstoffatomen von Y und/oder R¹ und/oder R² unter Bildung eines nicht-aromatischen Systems miteinander verbunden sein können, durch Umsetzung eines Lactons der Formel (II) 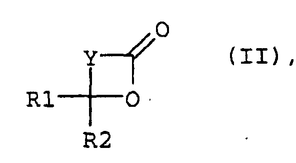 in der R¹, R² und Y die vorstehend genannte Bedeutung haben, mit Chlorwasserstoff und Phosgen in Gegenwart eines Katalysators gefunden, das dadurch gekennzeichnet ist, daß man Chlorwasserstoff vor und/oder während der Zugabe von Phosgen zuführt, mit der Einleitung des Chlorwasserstoffs erst bei einer Temperatur von mindestens 60°C beginnt und als Katalysator eine Pyridin-Verbindung einsetzt.

Beim erfindungsgemäßen Verfahren wird als Katalysator eine Pyridin-Verbindung der allgemeinen Formel (III) 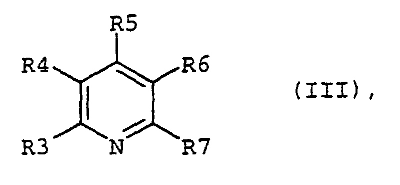 eingesetzt, in der die Reste R³ bis R⁷ unabhängig voneinander Wasserstoff, einen Kohlenstoff enthaltenden organischen Rest, Halogen, eine Nitro- oder eine Cyanogruppe bedeuten.

Die unsubstituierten oder substituierten, aliphatischen, aromatischen oder araliphatischen Reste mit 1 bis 20 Kohlenstoffatomen können ein oder mehrere Heteroatome, wie etwa Sauerstoff, Stickstoff oder Schwefel enthalten, beispielsweise -O-, -S-, -NR-, -CO- und/oder -N= in aliphatischen oder aromatischen Systemen, und/oder durch eine oder mehrere funktionelle Gruppen, welche beispielsweise Sauerstoff, Stickstoff, Schwefel und/oder Halogen enthalten, substituiert sein, wie beispielsweise durch Fluor, Chlor, Brom, Iod und/oder eine Cyanogruppe. Enthält der Kohlenstoff enthaltende organische Rest ein oder mehrere Heteroatome, so kann dieser auch über ein Heteroatom gebunden sein. Somit sind beispielsweise auch Ether-, Thioether- und tertiäre Aminogruppen eingeschlossen. Als bevorzugte Beispiele des Kohlenstoff enthaltenden organischen Rests seien C₁- bis C₂₀-Alkyl, insbesondere C₁- bis C₆-Alkyl, C₆- bis C₁₀-Aryl, C₇- bis C₂₀-Aralkyl, insbesondere C₇- bis C₁₀-Aralkyl, und C₇- bis C₂₀-Alkaryl, insbesondere C₇- bis C₁₀-Alkaryl, genannt.

Es ist ferner möglich, daß zwei benachbarte Reste unter Bildung eines nichtaromatischen oder aromatischen Systems miteinander verbunden sind.

Als Halogene seien Fluor, Chlor, Brom und Iod genannt.

Bevorzugt sind Pyridin-Verbindungen (III), bei denen R³ bis R⁷ unabhängig voneinander Wasserstoff, C₁- bis C₆-Alkyl, C₆- bis C₁₀-Aryl, C₇- bis C₁₀-Aralkyl oder C₇- bis C₁₀-Alkaryl bedeuten, beispielsweise Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Phenyl, 2-Methylphenyl (o-Tolyl), 3-Methylphenyl (m-Tolyl), 4-Methylphenyl (p-Tolyl), Naphthyl oder Benzyl.

Besonders bevorzugt sind Wasserstoff und C₁- bis C₄-Alkyl, wie beispielsweise Methyl, Ethyl, Propyl, 1-Methylethyl, Butyl, 1-Methyl-propyl, 2-Methylpropyl und 1,1-Dimethylethyl, insbesondere Wasserstoff und Methyl.

Beim erfindungsgemäßen Verfahren können als Pyridin-Verbindungen beispielsweise Pyridin, 2-Methylpyridin (α-Picolin), 3-Methylpyridin (β-Picolin), 4-Methylpyridin (γ-Picolin), 2,3-Dimethylpyridin, 2,4-Dimethylpyridin, 2,5-Dimethylpyridin, 2,6-Dimethylpyridin, 3,4-Dimethylpyridin, 3,5-Dimethylpyridin, 2,3,4-Trimethylpyridin, 2,3,5-Trimethylpyridin, 2,3,6-Trimethylpyridin, 2,4,6-Trimethylpyridin, 3,4,5-Trimethylpyridin, 2,3,4,5-Tetramethylpyridin, 2,3,4,6-Tetramethylpyridin, 2,3,5,6-Tetramethylpyridin, 2,3,4,5,6-Pentamethylpyridin, 2-Ethylpyridin, 3-Ethylpyridin, 4-Ethylpyridin, 2-Propylpyridin, 3-Propylpyridin, 4-Propylpyridin, 2-Butylpyridin, 3-Butylpyridin, 4-Butylpyridin, 2-Butylpyridin, 3-Butylpyridin, 4-Butylpyridin, 2-Phenylpyridin, 3-Phenylpyridin, 4-Phenylpyridin, Chinolin und Isochinolin eingesetzt werden.

Bevorzugt sind Pyridin und die mono-C₁-C₄-alkylpyridine, ganz besonders bevorzugt die Monomethylpyridine 2-Methylpyridin (α-Picolin), 3-Methylpyridin (β-Picolin) und 4-Methylpyridin (γ-Picolin), insbesondere 3-Methylpyridin (β-Picolin).

Die Pyridin-Verbindung (III) wird beim erfindungsgemäßen Verfahren im allgemeinen in einer Konzentration von 0,1 bis 20 mol-%, bevorzugt 0,1 bis 10 mol-%, besonders bevorzugt 0,5 bis 10 mol-%, insbesondere 1 bis 6 mol-%, bezogen auf das Lacton (II), eingesetzt.

Des weiteren ist beim erfindungsgemäßen Verfahren die Zufuhr von Chlorwasserstoff vor und/oder während der Zugabe von Phosgen wesentlich. Entscheidend für den Erfolg bei der Zugabe von Chlorwasserstoff ist die Temperatur, bei der mit der Einleitung begonnen wird. Beim erfindungsgemäßen Verfahren erfolgt die Einleitung von Chlorwasserstoff erst bei einer Temperatur der Reaktionsansatzes von mindestens 60°C. Bevorzugt erfolgt die Einleitung von Chlorwasserstoff erst bei einer Temperatur von mindestens 80°C, besonders bevorzugt 100°C und ganz besonders bevorzugt von mindestens 110°C, insbesondere von mindestens 120°C.

Mit steigender Starttemperatur der Chlorwasserstoff-Einleitung sinkt im Regelfall der Anteil oligomerer und polymerer Nebenprodukte. Die erreichbare Produktausbeute wird somit erhöht und die zu entsorgende Menge an Rückstand verringert.

Bei einer Temperatur unterhalb von 60°C, d.h. außerhalb des erfindungsgemäßen Bereichs, wird der Reaktionsansatz in der Regel zähflüssig oder sogar fest und die gewünschte Chlorierungsreaktion kommt zum Erliegen.

Die Zugabe des Chlorwasserstoffs erfolgt in der Regel gasförmig und kann (i) vor der Zugabe des Phosgens, (ii) vor und während der Zugabe des Phosgens oder (iii) während der Zugabe des Phosgens erfolgen. Bevorzugt sind die Varianten (ii) "vor und während" sowie (iii) "während" der Zugabe des Phosgens.

Wird vor oder während der Umsetzung mit Phosgen kein Chlorwasserstoff zugegeben oder erst nach der begonnenen Zufuhr von Phosgen zugeführt, so sinkt die erzielbare Ausbeute deutlich ab, wobei das Risiko eines zähflüssigen oder gar sich verfestigenden Reaktorinhalts signifikant steigt. Der negative Effekt wird in der Regel umso größer, je mehr Phosgen bereits zugegeben wurde.

Selbstverständlich kann auch nach Beendigung der Phosgen-Zugabe noch weiterhin Chlorwasserstoff eingeleitet werden. Dies ist gegebenenfalls zur Vervollständigung der Umsetzung als sogenannte Nachreaktion sogar vorteilhaft. Zudem wird nicht-umgesetztes, überschüssiges Phosgen aus der Reaktionslösung ausgetragen.

Die insgesamt zugebene Menge an Chlorwasserstoff ist im allgemeinen von untergeordneter Bedeutung. Typischerweise gibt man insgesamt 0,5 bis 2 mol, bevorzugt 0,5 bis 1,5 mol Chlorwasserstoff pro mol Lacton (II) hinzu.

Die Umsetzung mit dem Phosgen kann beim erfindungsgemäßen Verfahren bei einer Temperatur von 60 bis 200°C, bevorzugt 100 bis 200°C, besonders bevorzugt 110 bis 150°C durchgeführt werden. Sie erfolgt im allgemeinen bei einem Druck von 0,01 bis 5 MPa abs, bevorzugt 0,05 bis 0,2 MPa abs und besonders bevorzugt bei Atmosphärendruck. Phosgen kann dabei gasförmig und/oder flüssig zudosiert werden. Bevorzugt ist die Zugabe von gasförmigen Phosgen.

Die beim erfindungsgemäßen Verfahren insgesamt zugeführte Menge an Phosgen beträgt im allgemeinen 0,8 bis 1,5 mol, bevorzugt 0,9 bis 1,2 mol pro mol Lacton (II).

Die beim erfindungsgemäßen Verfahren herstellbaren Chlorcarbonsäurechloride besitzen die Formel (I) 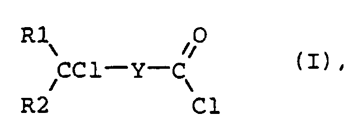 in der R¹ und R² unabhängig voneinander ein Wasserstoffatom, ein unsubstituierter oder substituierter, aliphatischer, aromatischer oder araliphatischer Rest mit 1 bis 20 Kohlenstoffatomen, ein Halogen, eine Nitro- oder eine Cyanogruppe bedeuten.

Der unsubstituierter oder substituierter, aliphatischer, aromatischer oder araliphatischer Rest mit 1 bis 20 Kohlenstoffatomen ist wie bei der Beschreibung der Pyridin-Verbindung (III) definiert. Als bevorzugte Beispiele des Kohlenstoff enthaltenden organischen Rests seien C₁- bis C₂₀-Alkyl, insbesondere C₁- bis C₆-Alkyl, C₆- bis C₁₀-Aryl, C₇- bis C₂₀-Aralkyl, insbesondere C₇- bis C₁₀-Aralkyl, und C₇- bis C₂₀-Alkaryl, insbesondere C₇- bis C₁₀-Alkaryl, genannt.

Als Halogene seien Fluor, Chlor, Brom und Iod genannt.

Bevorzugt sind die Chlorcarbonsäurechloride (I), bei denen R¹ und R² unabhängig voneinander Wasserstoff, C₁- bis C₆-Alkyl, C₆- bis C₁₀-Aryl, C₇- bis C₁₀-Aralkyl oder C₇- bis C₁₀-Alkaryl bedeuten, beispielsweise Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Phenyl, 2-Methylphenyl (o-Tolyl), 3-Methylphenyl (m-Tolyl), 4-Methylphenyl (p-Tolyl), Naphthyl oder Benzyl. Besonders bevorzugt sind Wasserstoff und C₁- bis C₄-Alkyl, insbesondere Wasserstoff.

Y bedeutet eine unsubstituierte oder durch Kohlenstoff enthaltende organische Reste, Halogen, Nitro- und/oder Cyanogruppen substituierte Alkylenkette mit 1 bis 10 Kohlenstoffatomen in der Kette, wobei die Alkylenkette durch eine Ether- (-O-), Thioether-(-S-), tertiäre Amino- (-NR-) oder Ketogruppe (-CO-) unterbrochen sein kann. Die Kohlenstoff enthaltenden organischen Reste und Halogen sind wie oben definiert.

Als Beispiele für den Rest Y seien die Alkylene (CH₂)ₙ mit n gleich 1 bis 10 genannt, wobei ein oder mehrere, gegebenenfalls alle Wasserstoffatome durch C₁- bis C₆-Alkyl, C₆- bis C₁₀-Aryl, C₇- bis C₁₀-Aralkyl und/oder C₇- bis C₁₀-Alkaryl, beispielsweise Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Phenyl, 2-Methylphenyl (O-Tolyl), 3-Methylphenyl (m-Tolyl), 4-Methylphenyl (p-Tolyl), Naphthyl oder Benzyl ersetzt sein können.

Es ist auch möglich, daß die organischen Reste R¹ und/oder R² und/oder von Y unter Bildung eines nicht-aromatischen Systems miteinander verbunden sind. Als Beispiel hierzu sei Hexahydrophthalid genannt.

Bevorzugt sind die Chlorcarbonsäurechloride (I), bei denen Y eine unsubstituierte oder durch Kohlenstoff enthaltende organische Reste, Halogen, Nitro- und/oder Cyanogruppen substituierte Alkylenkette mit 2 bis 8 und besonders bevorzugt 2 bis 4 Kohlenstoffatomen in der Kette bedeutet.

Ganz besonders bevorzugt sind die Chlorcarbonsäurechloride (I), bei denen die unsubstituierte oder substituierte Alkylenkette 3 Kohlenstoffatome in der Kette enthält, da gerade die 6-Ring-Lactone gegenüber ihren niedrigeren und höheren Homologen deutlich labiler sind und unter den Phosgenierungsbedingungen verstärkt zur Bildung oligomerer und polymerer Nebenprodukte neigen. Die beim erfindungsgemäßen Verfahren ganz besonders bevorzugten Chlorcarbonsäurechloride (I) sind somit 5-Chlorvaleriansäurechlorid (5-Chlorpentansäurechlorid) und dessen Derivate, insbesondere 5-Chlorvaleriansäurechlorid.

Die einzusetzenden Lactone besitzen die Formel (II) 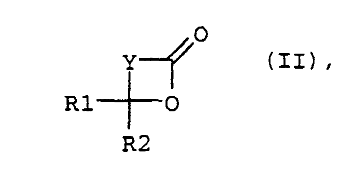 in der R¹, R² und Y die vorstehend genannten Bedeutungen haben. Selbstverständlich können auch Mischungen verschiedener Lactone eingesetzt werden. Ganz besonders bevorzugt eingesetzt werden δ-Valerolacton und dessen Derivate, insbesondere δ-Valerolacton.

Als Reaktoren für die Phosgenierung können prinzipiell die in der einschlägigen Fachliteratur beschriebenen Apparate für gas/flüssig- sowie flüssig/flüssig-Umsetzungen eingesetzt werden. Zur Erzielung einer hohen Raum/Zeit-Ausbeute ist eine intensive Durchmischung zwischen der Lacton (II) und Pyridin-Verbindung (III) enthaltende Lösung und dem zugegebenen Phosgen wichtig. Als nicht-einschränkende Beispiele seien genannt Rührkessel, Rührkesselkaskade, im Gegenstrom betriebene Reaktionskolonnen, Strömungsrohre (bevorzugt mit Einbauten), Blasensäulen und Schlaufenreaktoren.

Das Verfahren wird vorzugsweise ohne Lösungsmittel durchgeführt. Es ist jedoch möglich, ein gegen das eingesetzte Phosgen inertes Lösungsmittel zuzusetzen. Inerte Lösungsmittel sind beispielsweise aromatische Kohlenwasserstoffe, wie Toluol, Chlorbenzol, o-, m- oder p-Dichlorbenzol, o-, m- oder p-Xylol, cyclische Carbonate, wie Ethylencarbonat oder Propylencarbonat, das entsprechende Chlorcarbonsäurechlorid-Zielprodukt oder deren Gemische. Werden Lösungsmittel eingesetzt, so wird bevorzugt das Chlorcarbonsäurechlorid-Zielprodukt verwendet. Der Zusatz eines Lösungsmittels kann beispielsweise beim Einsatz höhermolekularer, zähflüssiger oder unter Reaktionsbedingungen fester Lactone (II) von Vorteil sein.

Das erfindungsgemäße Verfahren kann diskontinuierlich oder kontinuierlich durchgeführt werden.
a) diskontinuierlich
   Bei der diskontinuierlichen Herstellung wird das Reaktionsgemisch, enthaltend das Lacton (II), die Pyridin-Verbindung (III) und gegebenenfalls ein Lösungsmittel im allgemeinen in einem Reaktionsapparat, beispielsweise einem Rührkessel, vorgelegt und intensiv vermischt. Nun wird das Reaktionsgemisch erwärmt, wobei bei einer Temperatur von mindestens 60°C mit der Einleitung von Chlorwasserstoff begonnen werden kann. Gegebenenfalls wird das Reaktionsgemisch zunächst ohne Zugabe von Chlorwasserstoffgas auf eine Temperatur von mindestens 100°C oder darüber erwärmt und anschließend Chlorwasserstoff und Phosgen beispielsweise zusammen zugegeben. Nach Beendigung der Zugabe an Phosgen läßt man die Reaktionslösung wenige Minuten bis wenige Stunden nachreagieren,
   wobei optional eine weitere Zufuhr von Chlorwasserstoff möglich ist. Die Nachreaktion kann im Reaktionsapparat oder auch in einem nachgeschalteten Gefäß erfolgen.
b) kontinuierlich
   Für das kontinuierliche Verfahren geeignete Reaktionsapparate sind beispielsweise Rührkessel, Rührkesselkaskaden oder im Gegenstrom betriebene Reaktionskolonnen. Beim Start des kontinuierlichen Verfahrens legt man im allgemeinen ein Lösungsmittel (z.B. das entsprechende Chlorcarbonsäurechlorid-Zielprodukt) und die Pyridin-Verbindung (III) vor, bringt das System auf die gewünschte Temperatur und gibt gasförmigen Chlorwasserstoff hinzu.
   Anschließend beginnt man bei einer Temperatur von mindestens 100°C mit der kontinuierlichen Einleitung von Lacton (II), welches im allgemeinen weitere Pyridin-Verbindung (III) enthält und gegebenenfalls in einem Lösungsmittel gelöst sein kann, und der kontinuierlichen Zufuhr des flüssigen oder gasförmigen Phosgens. Parallel dazu wird in der Regel weiterhin Chlorwasserstoff zugeführt. Nachdem sich der Reaktorinhalt zum Chlorcarbonsäurechlorid umgesetzt hat, gleicht man die Mengen an Lacton (II) und dem Phosgen derart an, daß beide im wesentlichen äquimolar zugeführt werden. Eine der zugefahrenen Menge entsprechende Menge des Reaktionsvolumens wird dem Reaktionsapparat, beispielsweise über eine Standhaltung oder durch einen Überlauf, entnommen.
   Vorzugsweise wird die Reaktionslösung zur Nachreaktion einem weiteren Gefäß zugeführt.
   Alternativ kann dem Lacton (II) in einem, dem Phosgenierungsreaktor vorgeschalteten Reaktionsapparat bei gegebenenfalls niedrigerer Temperatur, jedoch bei mindestens 60°C, separat Chlorwasserstoff zugegeben werden.

Es ist im allgemeinen vorteilhaft, nicht-umgesetztes Phosgen oder Chlorwasserstoff beispielsweise durch Hindurchleiten eines Gases, welches gegenüber der Reaktionslösung chemisch inert ist, wie z.B. Stickstoff, anschließend aus der Reaktionslösung auszutreiben ("Strippen").

Nicht-umgesetztes Phosgen, welches beispielsweise bereits während der Synthesestufe aus dem Reaktor entweicht und/oder welches durch nachträgliches "Strippen" ausgetrieben wird, kann vorteilhafterweise aufgefangen und erneut eingesetzt werden. Geeignete Auffangvorrichtungen sind beispielsweise Kühlfallen, in denen das Phosgen auskondensiert.

Die aus der Umsetzung zwischen dem Lacton (II) und dem Phosgen stammende Reaktionslösung kann nach den üblichen Methoden aufgearbeitet werden. Bevorzugt ist eine destillative Aufarbeitung, wobei das optionale "Strippen" vor oder in der Destillationskolonne erfolgen kann.

Es ist möglich und gegebenenfalls vorteilhaft, die bei der destillativen Aufarbeitung erhaltene Fraktion, welche die pyridin-Verbindung (III) enthält, ganz oder teilweise rückzuführen. Je nach Lage der Siedepunkte des Chlorcarbonsäurechlorids (I) und der Pyridin-Verbindung (III) kann die Pyridin-Verbindung (III) beispielsweise vor oder nach dem Wertprodukt über Kopf abgetrennt werden. Falls das Verfahren mit einer Rückführung der Pyridin-Verbindung (III) ausgeübt wird, ist es von Vorteil, zur Ausschleusung möglicher Nebenprodukte nur einen Teil rückzuführen und den anderen Teil durch eine Zugabe einer frischen Pyridin-Verbindung (III) zu ersetzen.

In einer bevorzugten Ausführungsform zur diskontinuierlichen Herstellung der Chlorcarbonsäurechloride (I) legt man die gesamte Menge des entsprechenden Lactons (II), die Pyridin-Verbindung (III) und gegebenenfalls ein Lösungsmittel (z.B. das entsprechende Chlorcarbonsäurechlorid-Zielprodukt) in einem Rührkessel vor. Das Reaktionssystem wird nun auf eine Temperatur von mindestens 100°C oder darüber gebracht und gleichzeitig bei Atmosphärendruck mit der Einleitung von Chlorwasserstoff und Phosgen begonnen. Alternativ kann auch zunächst mit der Einleitung von Chlorwasserstoff begonnen werden und erst anschließend Phosgen zugeführt werden. Die gebildeten Koppelprodukte Kohlendioxid und Chlorwasserstoff werden abgeführt. Nachdem die gewünschte Menge an Phosgen und parallel dazu Chlorwasserstoff zugeführt wurde, wird die Reaktionslösung noch einige Zeit bei der eingestellten Temperatur zur Nachreaktion belassen. Bei der Nachreaktion kann dabei optional noch weiterer Chlorwasserstoff eingeleitet werden. Um das überschüssige Phosgen und dessen Koppelprodukte Kohlendioxid und Chlorwasserstoff aus der Reaktionslösung zu entfernen beziehungsweise abzureichern, ist es möglich, anschließend Inertgas durchzuleiten ("Strippen"). Die erhaltene Reaktionslösung wird schließlich der Aufarbeitung zugeführt. Im allgemeinen erfolgt die Aufarbeitung destillativ, gegebenenfalls unter Vakuum. Bei hochmolekularen Chlorcarbonsäurechloriden sind auch andere Reinigungsverfahren, wie beispielsweise Kristallisation möglich.

In einer allgemeinen Ausführungsform zur kontinuierlichen Herstellung der Chlorcarbonsäurechloride (I) legt man im Reaktor, z.B. einem Rührkessel, ein Lösungsmittel (z.B. das entsprechende Chlorcarbonsäurechlorid-Zielprodukt) und die Pyridin-Verbindung (III) vor, bringt das System auf die gewünschte Temperatur von mindestens 60°C und gibt gasförmigen Chlorwasserstoff hinzu. Anschließend beginnt man bei einer Temperatur von mindestens 100°C mit der kontinuierlichen Einleitung von Lacton (II), welches im allgemeinen weitere Pyridin-Verbindung (III) enthält und gegebenenfalls in einem Lösungsmittel gelöst sein kann, und der kontinuierlichen Zufuhr des flüssigen oder gasförmigen Phosgens. Parallel dazu wird in der Regel weiterhin Chlorwasserstoff zugeführt. Nachdem sich der Reaktorinhalt zum Chlorcarbonsäurechlorid umgesetzt hat, gleicht man die Mengen an Lacton (II) und dem Phosgen derart an, daß beide im wesentlichen äquimolar zugeführt werden. Eine der zugefahrenen Menge entsprechende Menge des Reaktionsvolumens wird dem Reaktionsapparat, beispielsweise über eine Standhaltung oder durch einen Überlauf, entnommen.

Die entnommene Reaktionslösung wird in einem nachgeschalteten Behälter, beispielsweise einem Rührkessel, zur Nachreaktion aufgefangen. Bei der Nachreaktion kann dabei optional noch weiterer Chlorwasserstoff eingeleitet werden. Nachdem auch der nachgeschaltete Behälter durch den Reaktionsaustrag gefüllt wurde, wird der Überlauf gegebenenfalls von den Koppelprodukte Kohlendioxid und Chlorwasserstoff wie oben beschrieben befreit und der Aufarbeitung zugeführt. Die Aufarbeitung kann beispielsweise destillativ durchgeführt werden.

Das erfindungsgemäße Verfahren ermöglicht die Herstellung von Chlorcarbonsäurechloriden, insbesondere 5-Chlorvaleriansäurechloriden und deren Derivate durch Phosgenierung der entsprechenden Lactone in hoher Ausbeute und hoher Reinheit von über 98%. Die Bildung oligomerer und polymerer Nebenprodukte wird deutlich verringert, was neben der genannten hohen Ausbeute auch den Vorteil einer deutlich geringeren Menge an entsorgungsbedürftigen Rückstand mit sich bringt. Da der Reaktionsansatz nicht mehr oder nur noch in stark vermindertem Maße zu einer Erhöhung der Viskosität neigt, ist auch das Sicherheitsrisiko durch Verstopfung von Apparaten und Leitungen im Bereich der Synthese und der Aufarbeitung deutlich minimiert. Eine sichere Reaktionsführung wird somit gewährleistet. Des weiteren wird auch die Neigung zur Schaumbildung während der Synthese erheblich verringert.

### Beispiele

### Beispiel 1 (erfindungsgemäß)

350 kg δ-Valerolacton (3,5 kmol) und 16 kg β-Picolin (3-Methylpyridin, 0,2 kmol) wurden in einem 630 1-Stahl-Email-Kessel vorgelegt und auf 120°C erhitzt. Bei 120°C wurde mit der Eingasung von 1 m³/h Chlorwasserstoff begonnen und die Temperatur weiter erhöht. Nach Erreichen von 140°C wurde mit der zusätzlichen Einleitung von gasförmigen Phosgen begonnen, wobei innerhalb von 24 Stunden insgesamt 380 kg Phosgen (3,84 kmol) eingegast wurden. Die Temperatur stiegt dabei auf 145°C an. Nach beendeter Phosgenzufuhr wurde noch zur Nachreaktion für 3 weitere Stunden Chlorwasserstoff zugeführt. Anschließend wurde auf 80°C abgekühlt und 12 Stunden mit 2,5 m³/h Stickstoff überschüssiges Phosgen ausgestrippt. Vom Rohaustrag wurde bei 1 kPa abs (10 mbar abs) und 85°C Kopftemperatur zunächst ein Vorlauf von ca. 20 l abgezogen und dann fraktioniert destilliert. Es wurden 440 kg 5-Chlorvaleriansäurechlorid (2,84 kmol) mit einer Reinheit von > 98 GC-Flächen-% erhalten. Dies entspricht einer Ausbeute von 81%.

### Versuchsanordnung für die Beispiele 2 bis 8

Die Versuchsanordnung umfasste ein 1 l Doppelmantel-Glasgefäß mit einem Rührer, einer Thermostatierung, je einem Einleitungsrohr für den gasförmigen Chlorwasserstoff und das Phosgen und einer zweiteiligen Kühlerkaskade. Die zweiteilige Kühlerkaskade umfasste einen Intensivkühler, welcher auf -10°C temperiert wurde und einen Kohlensäurekühler, welcher auf -78°C temperiert wurde. Die Versuche wurden bei Atmosphärendruck durchgeführt.

### Beispiel 2 (Vergleichsbeispiel)

200 g δ-Valerolacton (2 mol) und 8,1 g 3-Methylpyridin (β-Picolin, 0,1 mol) wurden unter Rühren langsam erhitzt. Bei einer Temperatur von 50°C wurde mit der Einleitung von 10 Nl/h Chlorwasserstoff begonnen und die Temperatur weiter erhöht. Nach Erreichen von 145°C wurde mit der zusätzlichen Einleitung von Phosgen begonnen. Bei 145 bis 150°C wurden innerhalb von 2 Stunden insgesamt 46 g Chlorwasserstoff (1,26 mol) und 102 g Phosgen (1,03 mol) eingegast. Dabei wurde ein erheblicher Viskositätsanstieg beobachtet. Um ein Abbrechen des Glasrührers zu vermeiden mußte der Versuch abgebrochen werden.

### Beispiel 3 (erfindungsgemäß)

200 g δ-Valerolacton (2 mol) und 8,1 g β-Picolin (3-Methylpyridin, 0,1 mol) wurden unter Rühren langsam erhitzt. Bei einer Temperatur von 80°C wurde mit der Einleitung von 10 Nl/h Chlorwasserstoff begonnen und die Temperatur weiter erhöht. Nach Erreichen von 140°C wurde mit der zusätzlichen Einleitung von Phosgen begonnen. Bei 140 bis 150°C wurden innerhalb von 4 Stunden und 45 Minuten insgesamt 60 g Chlorwasserstoff (1,64 mol) und 193 g Phosgen (1,95 mol) eingegast. Nach Beendigung der Gaszufuhr wurde das Reaktionsgemisch unter weiterem Rühren 1 Stunde bei 145°C zur Nachreaktion belassen. Anschließend wurde überschüssiges Phosgen durch Hindurchleiten von Stickstoff ausgestrippt und der Ansatz fraktioniert destilliert. Es wurden 205 g 5-Chlorvaleriansäurechlorid (1,32 mol) mit einer Reinheit von > 98 GC-Flächen-% erhalten. Dies entspricht einer Ausbeute von 66%.

### Beispiel 4 (erfindungsgemäß)

200 g δ-Valerolacton (2 mol) und 8,1 g β-Picolin (3-Methylpyridin, 0,1 mol) wurden unter Rühren langsam erhitzt. Bei einer Temperatur von 110°C wurde mit der Einleitung von 10 Nl/h Chlorwasserstoff begonnen und die Temperatur weiter erhöht. Nach Erreichen von 142°C wurde mit der zusätzlichen Einleitung von Phosgen begonnen. Bei 142 bis 150°C wurden innerhalb von 4 Stunden und 15 Minuten insgesamt 70 g Chlorwasserstoff (1,92 mol) und 202 g Phosgen (2,04 mol) eingegast. Nach Beendigung der Gaszufuhr wurde das Reaktionsgemisch unter weiterem Rühren 1 Stunde bei 145°C zur Nachreaktion belassen. Anschließend wurde überschüssiges Phosgen durch Hindurchleiten von Stickstoff ausgestrippt und der Ansatz fraktioniert destilliert. Es wurden 250 g 5-Chlorvaleriansäurechlorid (1,61 mol) mit einer Reinheit von > 98 GC-Flächen-% erhalten. Dies entspricht einer Ausbeute von 81%.

### Beispiel 5 (erfindungsgemäß)

200 g δ-Valerolacton (2 mol) und 8,1 g β-Picolin (3-Methylpyridin, 0,1 mol) wurden unter Rühren langsam erhitzt. Bei einer Temperatur von 138°C wurde gleichzeitig mit der Einleitung Chlorwasserstoff und Phosgen begonnen. Bei 138 bis 150°C wurden innerhalb von 3 Stunden und 15 Minuten insgesamt 61 g Chlorwasserstoff (1,67 mol) und 207 g Phosgen (2,09 mol) eingegast. Nach Beendigung der Gaszufuhr wurde das Reaktionsgemisch unter weiterem Rühren 1 Stunde bei 145°C zur Nachreaktion belassen. Anschließend wurde überschüssiges Phosgen durch Hindurchleiten von Stickstoff ausgestrippt und der Ansatz fraktioniert destilliert. Es wurden 270 g 5-Chlorvaleriansäurechlorid (1,74 mol) mit einer Reinheit von > 98 GC-Flächen-% erhalten. Dies entspricht einer Ausbeute von 87%.

### Beispiel 6 (Vergleichsbeispiel)

200 g δ-Valerolacton (2 mol) und 34,8 g Cyanex® 923 (Handelsprodukt von Fa. Cytec Industries, Gemisch verschiedener Trialkylphosphinoxide mit einem durchschnittlichen Molekulargewicht von 348 g/mol, 0,1 mol) wurden unter Rühren langsam erhitzt. Bei einer Temperatur von 120°C wurde mit der Einleitung von 10 Nl/h Chlorwasserstoff begonnen und die Temperatur weiter erhöht. Nach Erreichen von 146°C wurde mit der zusätzlichen Einleitung von Phosgen begonnen. Bei 146 bis 150°C wurden innerhalb von 3 Stunden insgesamt 44 g Chlorwasserstoff (1,21 mol) und 90 g Phosgen (0,91 mol) eingegast. Dabei wurde ein erheblicher Viskositätsanstieg beobachtet. Um ein Abbrechen des Glasrührers zu vermeiden mußte der versuch abgebrochen werden.

### Beispiel 7 (Vergleichsbeispiel)

200 g δ-Valerolacton (2 mol) und 12,8 g Dimethylpropylenharnstoff (1,3-Dimethyltetrahydro-2(1H)-pyrimidinon, 0,1 mol) wurden unter Rühren langsam erhitzt. Bei einer Temperatur von 100°C wurde mit der Einleitung von 10 Nl/h Chlorwasserstoff begonnen und die Temperatur weiter erhöht. Nach Erreichen von 145°C wurde mit der zusätzlichen Einleitung von Phosgen begonnen. Bei 145 bis 150°C wurden innerhalb von 25 Minuten insgesamt 6 g Chlorwasserstoff (0,16 mol) und 7 g Phosgen (0,07 mol) eingegast. Dabei wurde ein erheblicher Viskositätsanstieg beobachtet. Um ein Abbrechen des Glasrührers zu vermeiden mußte der Versuch abgebrochen werden.

### Beispiel 8 (Vergleichsbeispiel)

200 g δ-Valerolacton (2 mol) und 12,8 g Dimethylpropylenharnstoff (1,3-Dimethyltetrahydro-2(1H)-pyrimidinon, 0,1 mol) wurden unter Rühren langsam erhitzt. Bei einer Temperatur von 138°C wurde gleichzeitig mit der Einleitung Chlorwasserstoff und Phosgen begonnen. Bei 145 bis 150°C wurden innerhalb von 3 Stunden insgesamt 67 g Chlorwasserstoff (1,84 mol) und 210 g Phosgen (2,12 mol) eingegast. Nach Beendigung der Gaszufuhr wurde das Reaktionsgemisch unter weiterem Rühren 1 Stunde bei 145°C zur Nachreaktion belassen. Anschließend wurde überschüssiges Phosgen durch Hindurchleiten von Stickstoff ausgestrippt und der Ansatz fraktioniert destilliert. Es wurden 248 g 5-Chlorvaleriansäurechlorid (1,60 mol) mit einer Reinheit von > 98 GC-Flächen-% erhalten. Dies entspricht einer Ausbeute von 80%.

### Beispiel 9 (Vergleichsbeispiel)

192 g γ-Butyrolacton (2,23 mol) und 2 g Pyridin (0,025 mol) wurden im Doppelmantel-Glasgefäß vorgelegt und auf 120°C erwärmt. Unter intensivem Rühren wurden bei 120 bis 124°C innerhalb von 8 Stunden insgesamt 60 g gasförmiges Phosgen (0,61 mol) eingeleitet. Nach Herausstrippen des restlichen, nicht-umgesetzten Phosgens mit Stickstoff wurde der Rohaustrag fraktioniert destilliert. Die erste Fraktion mit 76 g enthielt 21,6 GC-Flächen-% 4-Chlorbuttersäurechlorid, die zweite Fraktion mit 110 g 2,6 GC-Flächen-% 4-Chlorbuttersäurechlorid. Dies entspricht einer Gesamtausbeute von 6%.

In Tabelle 1 ist eine Zusammenfassung der experimentellen Daten dargestellt. Beispiel 2 zeigt, daß bei einem Einleitungsbeginn des Chlorwasserstoffs unterhalb der erfindungsgemäßen Temperaturgrenze die Reaktion aufgrund eines deutlichen Viskositätsanstiegs abgebrochen werden mußte. Anhand der erfindungsgemäßen Beispiele 3 bis 5 geht der deutliche Anstieg der Ausbeute an 5-Chlorvaleriansäurechlorid mit steigender Starttemperatur der Chlorwasserstoff-Einleitung hervor. Bei einem Beginn der Chlorwasserstoff-Einleitung von 138°C konnte eine Ausbeute von 87% realisiert werden.

Während beim erfindungsgemäßen Beispiel 4 mit β-Picolin als Katalysator und einer beginnenden Chlorwasserstoff-Einleitung bei 110°C eine Ausbeute an 5-Chlorvaleriansäurechlorid von 81% erzielt werden konnte, führt der Einsatz von Trialkylphosphinoxiden und Dimethylpropylenharnstoff als Katalysator (siehe Vergleichsbeispiele 6 und 7) zu einem deutlichen Viskositätsanstieg der Reaktionslösung. In beiden Fällen mußte die Reaktion abgebrochen werden.

Das erfindungsgemäße Beispiel 3 zeigt daß beim Einsatz von β-Picolin auch bei einer Einleittemperatur des Chlorwasserstoffgases von deutlich unter 100°C ein flüssiger Reaktoraustrag erhalten wird, wohingegen beim Einsatz von Trialkylphosphinoxiden und Dimethylpropylenharnstoff als Katalysator (siehe Vergleichsbeispiele 6 und 7) bereits bei 120°C beziehungsweise 100°C die Reaktion aufgrund eines zähflüssigen Reaktorinhaltes abgebrochen werden mußte.

Wie der vergleich zwischen dem erfindungsgemäßen Beispiel 5 und dem Vergleichsbeispiel 8 zeigt, kann zwar auch beim nicht-erfindungsgemäßen Einsatz von Katalysatoren, wie Dimethylpropylenharnstoff, durch Erhöhung der Starttemperatur für die Chlorwasserstoff-Einleitung eine gewisse Verbesserung erzielt, jedoch bei weitem nicht das hervorragende Ergebnis des erfindungsgemäßen Verfahrens erreicht werden. Das erfindungsgemäße Beispiel 5 führte unter sonst vergleichbaren Bedingungen zu einer ca. 9 rel.-% höheren Ausbeute.

Selbst wenn der nicht-erfindungsgemäße Einsatz von Katalysatoren, wie Dimethylpropylenharnstoff, bei einer höheren Starttemperatur von 138°C für die Chlorwasserstoff-Einleitung einen flüssigen Reaktoraustrag ermöglicht, wie Vergleichsbeispiel 8 zeigt, besteht bei einem Ausfall der Reaktorbeheizung das Risiko der Schaumbildung und des Viskositätsanstiegs bis hin zur Verfestigung des Reaktorinhaltes. Der erfindungsgemäße Einsatz der Pyridin-Verbindungen (III) ist dem nicht-erfindungsgemäße Einsatz von Katalysatoren, wie beispielsweise Harnstoffverbindungen oder Phosphinoxiden überlegen.

Das Vergleichsbeispiel 9 zeigt, daß selbst beim Einsatz von Pyridin-Verbindungen (III) ohne die Einleitung von Chlorwasserstoff nur eine unzureichende Ausbeute erzielt wird.

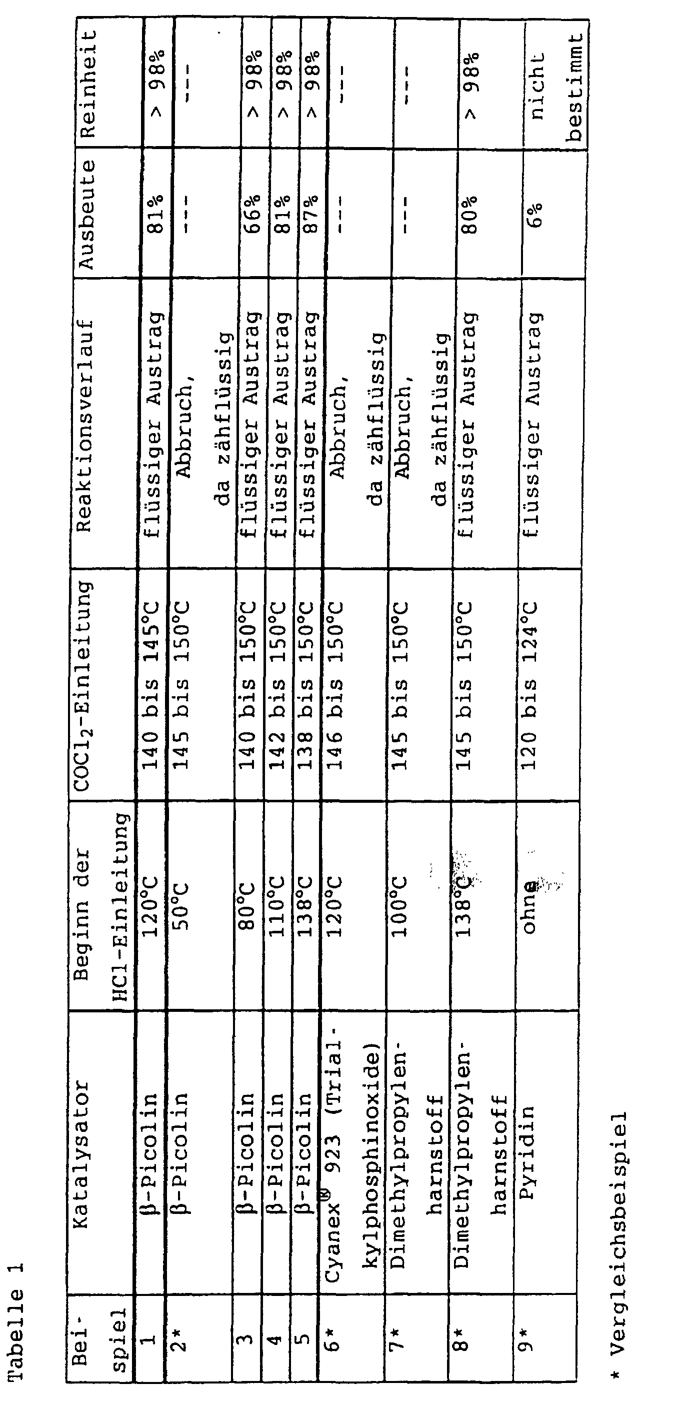

## Patentansprüche

1. Verfahren zur Herstellung von Chlorcarbonsäurechloriden der Formel (I) 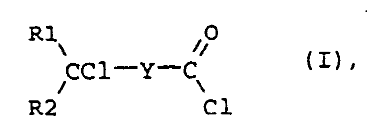 in der
R¹ und R² unabhängig voneinander
ein Wasserstoffatom, ein unsubstituierter oder substituierter, aliphatischer, aromatischer oder araliphatischer Rest mit 1 bis 20 Kohlenstoffatomen, ein Halogen, eine Nitro- oder eine Cyanogruppe bedeuten,
und Y
eine Alkylenkette mit 1 bis 10 Kohlenstoffatomen in der Kette, die unsubstituiert oder durch unsubstituierte oder substituierte, aliphatische, aromatische oder araliphatische Reste mit 1 bis 20 Kohlenstoffatomen, Halogen, Nitro- und/oder Cyanogruppen substituiert ist, wobei die Alkylenkette durch eine Ether-, Thioether-, tertiäre Amino- oder Ketogruppe unterbrochen sein kann,
bedeutet,
wobei die genannten unsubstituierten oder substituierten, aliphatischen, aromatischen oder araliphatischen Reste mit 1 bis 20 Kohlenstoffatomen von Y und/oder R¹ und/oder R² unter Bildung eines nicht-aromatischen Systems miteinander verbunden sein können, durch Umsetzung eines Lactons der Formel (II) 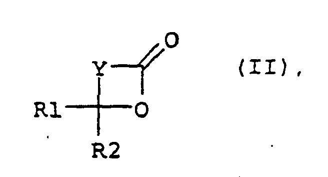 in der R¹, R² und Y die vorstehend genannte Bedeutung haben, mit Chlorwasserstoff und Phosgen in Gegenwart eines Katalysators, **dadurch gekennzeichnet, daß** man Chlorwasserstoff vor und/oder während der Zugabe von Phosgen zuführt, mit der Einleitung des Chlorwasserstoffs erst bei einer Temperatur von mindestens 60°C beginnt und als Katalysator eine Pyridin-Verbindung einsetzt.

2. Verfahren nach Anspruch 1, **dadurch gekennzeichnet, daß** man den Katalysator in einer Konzentration von 0,1 bis 20 mol-% bezogen auf das Lacton (II) einsetzt.

3. Verfahren nach den Ansprüchen 1 bis 2, **dadurch gekennzeichnet, daß** man als Katalysator 3-Methylpyridin (β-Picolin) einsetzt.

4. Verfahren nach den Ansprüchen 1 bis 3, **dadurch gekennzeichnet, daß** man insgesamt 0,5 bis 2 mol Chlorwasserstoff pro mol Lacton (II) einsetzt.

5. Verfahren nach den Ansprüchen 1 bis 4, **dadurch gekennzeichnet, daß** man die Einleitung von Chlorwasserstoff erst bei einer Temperatur von mindestens 100°C beginnt.

6. Verfahren nach den Ansprüchen 1 bis 5, **dadurch gekennzeichnet, daß** man die Umsetzung mit dem Phosgen bei einer Temperatur von 100 bis 200°C und einem Druck von 0,01 bis 5 MPa abs durchführt.

7. Verfahren nach den Ansprüchen 1 bis 6, **dadurch gekennzeichnet, daß** man 5-Chlorvaleriansäurechlorid oder dessen Derivate herstellt.

8. Verfahren nach den Ansprüchen 1 bis 7, **dadurch gekennzeichnet, daß** man 5-Chlorvaleriansäurechlorid herstellt.

## Claims

1. A process for preparing chlorocarbonyl chlorides of the formula (I) 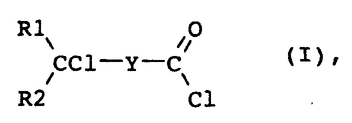 in which
R¹ and R² independently of one another
are a hydrogen atom, an unsubstituted or substituted, aliphatic, aromatic or araliphatic radical having 1 to 20 carbon atoms, a halogen, a nitro or a cyano group,
and Y
is an alkylene chain having 1 to 10 carbon atoms in the chain, which is unsubstituted or substituted by unsubstituted or substituted, aliphatic, aromatic or araliphatic radicals having 1 to 20 carbon atoms, halogen, nitro and/or cyano groups, where the alkylene chain may be interrupted by an ether, a thioether, a tertiary amino or a keto group,
where said unsubstituted or substituted, aliphatic, aromatic or araliphatic radicals having 1 to 20 carbon atoms of Y and/or R¹ and/or R² may be attached to one another forming a nonaromatic system, by reacting a lactone of the formula (II) 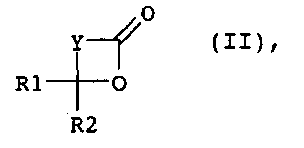 in which R¹, R² and Y are as defined above, with hydrogen chloride and phosgene in the presence of a catalyst, which comprises introducing hydrogen chloride before and/or during the addition of phosgene, where the introduction of hydrogen chloride is started only when a temperature of at least 60°C has been reached and a pyridine compound is used as catalyst.

2. A process as claimed in claim 1, wherein the catalyst is employed in a concentration of 0.1-20 mol%, based on the lactone (II).

3. A process as claimed in claim 1 or 2, wherein the catalyst used is 3-methylpyridine (β-picoline).

4. A process as claimed in any of claims 1 to 3, wherein a total of 0.5-2 mol of hydrogen chloride are employed per mole of lactone (II).

5. A process as claimed in any of claims 1 to 4, wherein the introduction of hydrogen chloride is started only when a temperature of at least 100°C has been reached.

6. A process as claimed in any of claims 1 to 5, wherein the reaction with phosgene is carried out at a temperature of 100-200°C and an absolute pressure of 0.01-5 MPa.

7. A process as claimed in any of claims 1 to 6, wherein 5-chlorovaleryl chloride or derivatives thereof are prepared.

8. A process as claimed in any of claims 1 to 7, wherein 5-chlorovaleryl chloride is prepared.

## Revendications

1. Procédé de préparation de chlorures d'acide chlorocarboxylique de la formule (I) 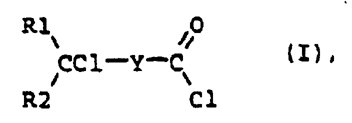 dans laquelle
R¹ et R² représentent indépendamment l'un de l'autre
un atome d'hydrogène, un radical aliphatique, aromatique ou araliphatique, substitué ou non substitué, comportant 1 à 20 atomes de carbone, un halogène, ou un groupe nitro ou cyano,
et Y
représente une chaîne alkylène comportant 1 à 10 atomes de carbone dans la chaîne, qui est non substituée ou substituée par des radicaux aliphatiques, aromatiques ou araliphatiques, substitués ou non substitués, comportant 1 à 20 atomes de carbone, de l'halogène ou des groupes nitro et/ou cyano, la chaîne alkylène pouvant être interrompue par un groupe éther, thioéther, amino tertiaire ou céto,
dans lequel les radicaux cités aliphatiques, aromatiques ou araliphatiques, substitués ou non substitués, comportant 1 à 20 atomes de carbone de Y et/ou de R¹ et/ou de R² peuvent être mutuellement reliés avec formation d'un système non aromatique, par réaction d'une lactone de la formule (II) 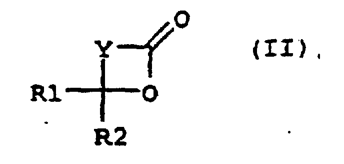 dans laquelle R¹, R² et Y ont la signification indiquée précédemment, avec du chlorure d'hydrogène et du phosgène en présence d'un catalyseur, **caractérisé en ce qu'**on amène le chlorure d'hydrogène avant et/ou pendant l'addition de phosgène, on commence avec l'introduction du chlorure d'hydrogène uniquement à une température d'au moins 60°C et on met en oeuvre comme catalyseur un composé de pyridine.

2. Procédé suivant la revendication 1, **caractérisé en ce qu'**on met en oeuvre le catalyseur en une concentration de 0,1 à 20 % molaire par rapport à la lactone (II).

3. Procédé suivant les revendications 1 et 2, **caractérisé en ce que**, comme catalyseur, on met en oeuvre de la 3-méthylpyridine (β-picoline).

4. Procédé suivant les revendications 1 à 3, **caractérisé en ce qu'**on met en oeuvre au total 0,5 à 2 moles de chlorure d'hydrogène par mole de lactone (II).

5. Procédé suivant les revendications 1 à 4, **caractérisé en ce qu'**on commence l'introduction de chlorure d'hydrogène uniquement à une température d'au moins 100 °C.

6. Procédé suivant les revendications 1 à 5, **caractérisé en ce qu'**on effectue la réaction avec le phosgène à une température de 100 à 200 °C et à une pression de 0,01 à 5 MPa abs.

7. Procédé suivant les revendications 1 à 6, **caractérisé en ce qu'**on prépare du chlorure d'acide 5-chlorovalérianique ou ses dérivés.

8. Procédé suivant les revendications 1 à 7, **caractérisé en ce qu'**on prépare du chlorure d'acide 5-chlorovalérianique.
